# EUROPÄISCHE PATENTSCHRIFT

(11) **EP 0 547 476 B1**
(45) Veröffentlichungstag und Bekanntmachung des Hinweises auf die Patenterteilung: **14.02.1996**
(21) Anmeldenummer: 92120890.6
(22) Anmeldetag: 08.12.1992
(51) Int. Cl.: A61F 2/38

(54) **Kniegelenkendoprothese**
Knee prosthesis
Prothèse du genou

(30) Priorität: 18.12.1991 DE 4141757
(43) Veröffentlichungstag der Anmeldung: 23.06.1993
(73) Patentinhaber: ESKA medical GmbH & Co., D-23556 Lübeck (DE)
(72) Erfinder: Grundei, Hans, W-2400 Lübeck (DE)
(74) Vertreter: Dr. Fuchs, Dr. Luderschmidt Dr. Mehler, Dipl.-Ing. Weiss Patentanwälte

(56) Entgegenhaltungen:
- DE-A- 2 607 316
- DE-A- 3 343 606
- FR-A- 2 245 327
- FR-A- 2 330 375
- FR-A- 2 566 657
- FR-A- 2 628 316
- US-A- 4 358 859
- Kopie eines Prospektes der London Splint Company Ltd.
- Kopie des Artikels "Das GT-Kniegelenksystem Lübeck - Biomechanische Gesichtspunkte, Konstruktion und Anwendung" aus ORTHOPÄDIE-TECHNIK, 34. Jahrgang 1983, Heft 12, Seiten 223-226

## Beschreibung

Es sind eine Fülle von künstlichen Kniegelenken bekannt geworden, die zum Ersatz des natürlichen Kniegelenkes im menschlichen Körper implantiert werden.

Grob lassen sich diese Endoprothesen in zwei Gruppen unterteilen. Die Gelenke der einen Gruppe finden Anwendung bei einem relativ intaktem Bandapparat und versuchen, aufgrund ihrer Konstruktion einen möglichst physiologischen Bewegungsablauf zu ermöglichen. Als Beispiel sei hier die Kniegelenkendoprothese gemäß der DE 39 22 294 C1 genannt.

Desweiteren sind Endoprothesen des Scharniertyps bekannt, bei denen das femorale und das tibiale Teil der Prothese über ein Scharniergelenk miteinander verbunden sind. Bei diesen Gelenken kann auf den Bandapparat verzichtet werden. Ihre Applikation erfolgt bevorzugt bei schwerst zerstörten Gelenken, bei denen es also nicht darauf ankommt, einen möglichst physiologischen Bewegungsablauf zu erzielen, sondern überhaupt erst eine Implantation eines künstlichen Gelenks zu ermöglichen. Problematisch bei den bekannten Scharnierprothesen ist allerdings die Stabilität. So muß bei üblichen Scharnierprothesen das Scharnier selbst alle auftretenden Belastungen, die beim Gehen auftreten, von der Tibia in den Femurknochen und umgekehrt leiten. Auch bei Lastwechseln ist das Scharniergelenk gefordert. Dies führt in der Praxis zu Stabilitätsproblemen, namentlich zu der Tendenz, daß das Scharnier ausschlägt.

Aufgabe der vorliegenden Erfindung ist es, eine Kniegelenkendoprothese zur Applikation bei schwerstzerstörten Gelenken anzugeben, die hinreichend stabil ist, also allen auftretenden Belastungen, einschließlich Lastwechseln, über einen langen Zeitraum zu widerstehen.

Gelöst wird diese Aufgabe durch die Kniegelenkendoprothese mit den Merkmalen des Anspruchs 1. Weitere vorteilhafte Ausgestaltungen ergeben sich aus den Unteransprüchen.

Demgemäß besteht die erfindungsgemäße Kniegelenkendoprothese aus einem mit einem im Femurknochen zu verankernden Stielteil verbindbaren Femurteil, das mit zwei Gleitkufen versehen ist, die ventral über eine ein Verbindungselement zum Stielteil tragenden Brücke miteinander verbunden sind und zwischen sich einen von ventral nach dorsal verlaufenden Schlitz begrenzen. Dieser ist beidseitig femurwärts von jeweils einem Steg eingefaßt, in dem jeweils eine von medial nach lateral verlaufende Auskehlung mit teilkreisförmigem Querschnitt vorgesehen ist. Beide Auskehlungen fluchten miteinander.

Des weiteren ist ein Tibiateil vorgesehen, welches zwei Gleitbahnen, auf denen die Gleitkufen des Femurteils eine Gleitbewegung ausführen können, einen zwischen den beiden Gleitbahnen femurwärts gerichteten Steg, der durch den Schlitz des Femurteils greift und eng an dessen Innenflächen anliegend geführt ist, sowie einen von medial nach lateral verlaufenden zylindrischen Führungsbolzen im oberen Bereich des Steges aufweist. Dieser Führungsbolzen liegt in den Auskehlungen der Stege im Femurteil. Er ist in dieser Lage durch ihn umschließende Teile des Stielteils gesichert.

Der Kraftfluß bei der erfindungsgemäßen Kniegelenkendoprothese erfolgt - mit Ausnahme bei Lastwechseln - direkt über die Gleitbahnen des Tibiateils und die Gleitkufen des Femurteils. Lastwechsel werden über die Innenseiten des Schlitzes im Femurteil sowie über den eng an diesen anliegenden und durch den Schlitz greifenden Steg des Tibiateils aufgenommen. Demnach besitzt der Führungsbolzen ausschließlich eine Führungsfunktion, um nämlich das Femurteil wie um eine Polachse gegenüber dem Tibiateil zu beugen. Hierin ist der entscheidende Vorteil gegenüber den herkömmlichen Scharnierprothesen zu sehen.

Wie bei diesen führt jeder Punkt der femoralen Gleitkufen um die Polachse, gebildet durch den Führungsbolzen, beim Beugen des Gelenks eine Kreisbewegung aus. Die Ortskurve jedes Punktes der Gleitkufen ist demnach eine Kreisbahn.

Aufgrund der Entkoppelung der Belastungsübertragung auf die Gleitbahnen einerseits und auf den Steg des Tibiateils andererseits wird eine hervorragende Langzeitstabilität erreicht.

Die umschließenden Teile des Stielteils sind notwendig, um den Führungsbolzen in den Auskehlungen des Femurteils zu halten. Die Teile können entsprechend ausgeformte Laschen des Stielteils sein. Die Verbindung des Femurteils zum Stielteil kann in bekannter Weise durch eine konische Verklemmung zwischen einem als Konus ausgebildeten Verbindungselement des Femurteils einerseits und durch eine konische Hülse im Stielteil andererseits erfolgen.

Das Femurteil besteht vorzugsweise aus Metall, wohingegen die es berührenden Teile des Tibiateils, nämlich die Gleitbahnen, die Stege und der Führungsbolzen Oberflächen aus abriebsarmem Kunststoff aufweisen.

Der Steg ist vorzugsweise durch eine Metalleinlage armiert, um die auftretenden Belastungen bei Lastwechseln sicher aufnehmen zu können ohne nachzugeben.

Auch der Führungsbolzen kann durch einen inneren Metallkern armiert sein, um seine Führungsfunktion über lange Zeit ausüben zu können.

Die Oberflächen der Gleitbahnen des Tibiateils und die Oberflächen des Steges sind vorzugsweise einstückig in einem einzigen Aufsatzstück ausgeformt, das mit dem im übrigen metallischen Tibiateil verbunden ist. Dies gestattet bei einem eventuellen notwendig werdenden Revisionseingriff nach übermäßigem Abrieb des Kunststoffs einen relativ einfachen Ersatz des Gleitpartners für die femoralen Gleitkufen.

Vorteilhaft ist die metallische Einlage des Steges einstückig mit den metallischen Teilen des Tibiateiles ausgebildet. Dadurch werden etwaige Probleme einer Verbindung zwischen dieser Metalleinlage und dem Tibiateil umgangen.

Damit der Führungsbolzen seine Führungsfunktion möglichst optimal ausübt, weisen die Auskehlungen im Femurteil vorzugsweise eine Tiefe auf, die im wesentlichen dem halben Durchmesser des Führungsbolzens entspricht.

Als weiteren Vorteil der erfindungsgemäßen Endoprothese wird angesehen, daß diese aus modularen Bestandteilen zusammengesetzt wird und dennoch eine hervorragende Stabilität gewährleistet. Aufgrund ihrer Konstruktion ist es möglich, die Endoprothese ohne jedwede Schraubverbindung zusammenzustellen, die sich im Laufe der Zeit lockern könnte.

Die Erfindung wird anhand eines Ausführungsbeispiels gemäß den Zeichnungen näher erläutert. Hierbei zeigt:
- Figur 1:: eine Seitenansicht des Femurteils,
- Figur 2:: eine Ansicht des Femurteils von dorsal gesehen,
- Figur 3: eine Aufsicht auf das Femurteil,
- Figur 4: eine Schnittansicht der Endoprothese in der Ebene des Steges des Tibiateils,
- Figur 5: eine Schnittansicht entlang der Linie IV-IV aus Figur 4,
- Figur 6:: das Femurteil und das Stielteil der Endoprothese in Seitenansicht und
- Figur 7:: die Unterseite des Stielteils in Aufsicht.

In den Figuren sind gleiche Teile mit den selben Bezugszeichen versehen.

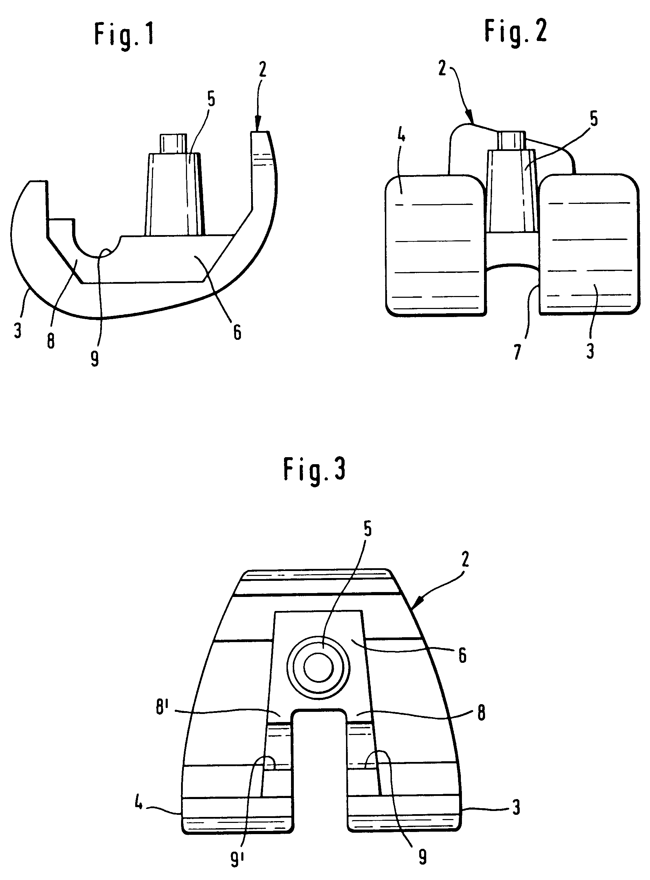

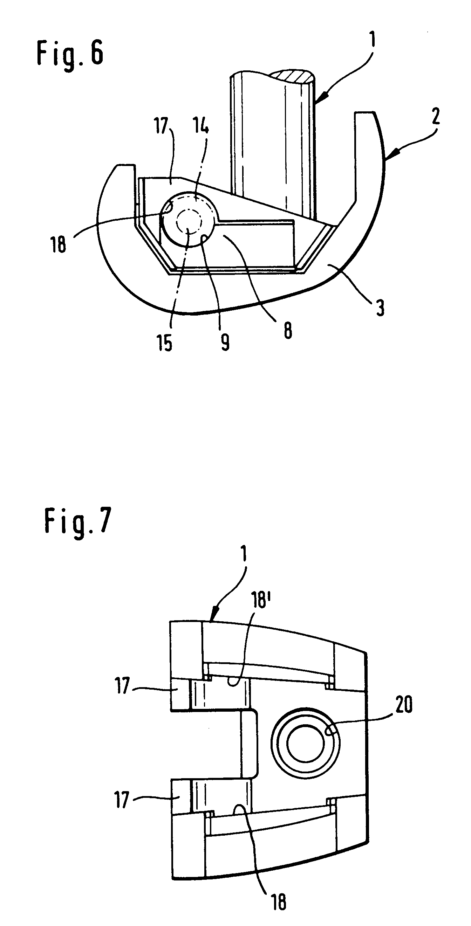

Figur 1 zeigt das mit einem im Femurknochen zu verankernden Stielteil verbindbaren Femurteil 2. Dieses weist zwei Gleitkufen 3 und 4 (Figur 2) auf, die ventral über eine Brücke 6 miteinander verbunden sind (Figur 3). Die Brücke 6 trägt darüber hinaus ein Verbindungselement 5 (Figur 1), welches im vorliegenden Falle als konischer Zapfen dargestellt ist. Dieser findet seine Entsprechung in einer konischen Hülse 16 (Figur 7) in dem Stielteil 1 (Figur 6). Zwischen dieser konischen Hülse 16 und dem Verbindungselement 5 wird in bekannter Weise eine Verbindung durch konische Verklemmung hergestellt. Die beiden Gleitkufen 3 und 4 des Femurteils 2 begrenzen zwischen sich einen von ventral nach dorsal verlaufenden Schlitz 7 (Figur 2). Dieser Schlitz 7 ist beidseitig femurwärts von jeweils einem Steg 8 bzw. 8' eingefaßt (Figur 3). In jedem der beiden Stege 8 und 8' ist eine medial nach lateral verlaufende Auskehlung 9 bzw. 9' mit teilkreisförmigem Querschnitt (Figur 1) vorgesehen. Beide Auskehlungen 9 bzw. 9' fluchten miteinander (Figur 3).

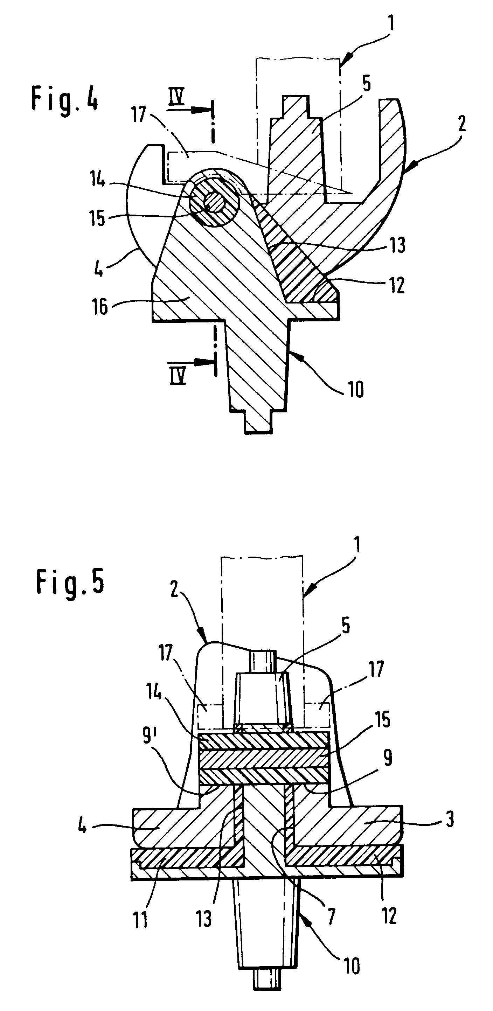

Das Zusammenwirken des Femurteils 2 mit dem Tibiateil 10 ist in den Figuren 4 und 5 verdeutlicht. Das Tibiateil 10 weist zwei Gleitbahnen 11, 12 auf, auf denen die Gleitkufen 3, 4 des Femurteils 2 eine Gleitbewegung ausführen können (Figur 5). Das Tibiateil 10 weist zwischen den beiden Gleitbahnen 11 und 12 einen femurwärts gerichteten Steg 13 auf, der durch den Schlitz 7 des Femurteils greift und eng an dessen Innenflächen anliegend geführt ist (Figur 5). Durch die enge Anlage des Steges an die Innenflächen des Schlitzes 7 werden die bei einem Lastwechsel auftretenden Kräfte mit geringem Spiel vom Femurteil 2 in das Tibiateil 10 geleitet.

Das Tibiateil 10 weist darüber hinaus einen von medial nach lateral verlaufenden zylindrischen Führungsbolzen 14 im oberen Bereich des Steges 13 auf (Figur 4 und 5), der in den Auskehlungen 9 und 9' der Stege 8 bzw. 8' im Femurteil 2 liegt. Der Führungsbolzen 14 bildet in seinem Lager der Auskehlungen 9 und 9' die Schwenk- oder Beugeachse des Gelenks. Er ist in seiner Lage durch ihn umschließende Teile 17 des Stielteils 1 (Figur 6) gesichert. Im dargestellten Ausführungsbeispiel sind die Teile 17 gebildet durch einen flächenförmigen Ansatz mit entsprechenden Ausformungen 18 und 18' (Figur 7), derart, daß der Führungsbolzen 14 durch die Auskehlungen 9 und 9' sowie den Ausformungen 18 und 18' in den Teilen 17 des Stielteils 1 praktisch rohrförmig umschlossen wird. Wie bereits ausgeführt, wird die Verbindung des Femurteils 2 an dem Stielteil 1 über eine konische Verklemmung vorgenommen.

Im dargestellten Ausführungsbeispiel ist das Tibiateil 10 als ein Teil dargestellt, welches zusätzlich noch mit einem (nicht dargestellten) Stielteil verbunden werden muß. Es kann freilich auch einstückig mit dem Stiel ausgebildet sein.

Wie insbesondere aus Figur 5 deutlich wird, sind die Teile des Tibiateils 10, welche mit dem Femurteil 2 in Berührung stehen, aus einem abriebsarmen Kunststoff gefertigt, was durch die unterschiedliche Schraffierung dieser Teile zum Ausdruck kommt. So bestehen die Gleitbahnen 11 und 12, die Oberflächen des Steges 13 sowie die Oberfläche des Führungsbolzens 14 aus einem derartigen Material. Aus Figur 5 wird darüber hinaus die bevorzugte Ausführungsform deutlich, wonach die Oberflächen des Steges 13 mit den Gleitbahnen 11 und 12 des Tibiateils 10 einstückig aus einem solchen Kunststoff gefertigt sind.

Wie ebenfalls aus der Figur 5 ersichtlich wird, weist der Steg 13 eine Metalleinlage 16 auf, durch welche er armiert ist. Diese Metalleinlage ist im dargestellten Ausführungsbeispiel einstückig mit den übrigen metallischen Teilen des Tibiateils ausgebildet (s.a. Figur 4).

Im übrigen ist im Ausführungsbeispiel der Führungsbolzen durch einen inneren Metallkern 15 armiert.

Aus Darstellungsgründen ist das Stielteil 1 in den Figuren 4 und 5 lediglich strichpunktiert dargestellt. Angedeutet aber ist, wie die Teile 17 zur Sicherung der Lage des Führungsbolzens 14 in den Auskehlungen 9 bzw. 9' dienen.

## Patentansprüche

1. Kniegelenkendoprothese bestehend aus
- einem mit einem im Femurknochen zu verankernden Stielteil (1) verbindbaren Femurteil (2), das mit zwei Gleitkufen (3, 4) versehen ist, die ventral über eine ein Verbindungselement (5) zum Stielteil (1) tragenden Brücke (6) miteinander verbunden sind und zwischen sich einen von ventral nach dorsal verlaufenden Schlitz (7) begrenzen, der beidseitig femurwärts von jeweils einem Steg (8 bzw. 8') eingefaßt ist, in dem jeweils eine von medial nach lateral verlaufende Auskehlung (9 bzw. 9') mit teilkreisförmigem Querschnitt vorgesehen ist, die beide miteinander fluchten, und
- einem Tibiateil (10), welches zwei Gleitbahnen (11, 12), auf denen die Gleitkufen (3, 4) des Femurteils (2) eine Gleitbewegung ausführen können, einen zwischen den beiden Gleitbahnen (11, 12) femurwärts gerichteten Steg (13), der durch den Schlitz (7) des Femurteils (2) greift und eng an dessen Innenflächen anliegend geführt ist, sowie einen von medial nach lateral verlaufenden zylindrischen Führungsbolzen (14) im oberen Bereich des Steges (13) aufweist, der in den Auskehlungen (9 bzw 9') der Stege (8) im Femurteil (2) liegt und in dieser Lage durch ihn umschließende Teile (17) des Stielteils (1) gesichert ist.

2. Kniegelenkendoprothese nach Anspruch 1, bei der das Femurteil (2) aus Metall besteht und die es berührenden Teile: Gleitbahnen (11, 12), Steg (13) und Führungsbolzen (14) des Tibiateils (10) Oberflächen aus abriebsarmen Kunststoff aufweisen.

3. Kniegelenkendoprothese nach Anspruch 1, bei der der Steg (13) durch eine Metalleinlage (16) armiert ist.

4. Kniegelenkendoprothese nach Anspruch 2 oder 3, bei der der Führungsbolzen (14) durch einen inneren Metallkern (15) armiert ist.

5. Kniegelenkendoprothese nach einem der Ansprüche 2 bis 4, bei der die Gleitbahnen (11, 12) und die Oberfläche des Steges (13) einstückig in einem einzigen Aufsatzstück ausgeformt sind, welches mit dem ansonsten metallischen Tibiateil (10) verbunden ist.

6. Kniegelenkendoprothese nach Anspruch 3, bei der die Metalleinlage (16) des Steges (13) einstückig mit den metallischen Teilen des Tibiteils (10) ausgebildet ist.

7. Knieendoprothese nach einem der Ansprüche 1 bis 6, bei der die Auskehlungen (9, 9') eine Tiefe (T) aufweisen, die im wesentlichen dem halben Durchmesser (D) des Führungsbolzens (14) entspricht.

## Claims

1. Knee joint endoprosthesis comprises
- a femoral part (2) which can be connected to a stem part (1) to be anchored in the femoral bone and which is provided with two runners (3, 4), which are connected to one another ventrally via a bridge (6) supporting a connecting element (5) for the stem part (1) and which define a slot (7) extending ventrally to dorsally between them, which is enclosed on both sides in the femoral direction by a web (8 or 8') in each case, in which is provided in each case a recess (9 or 9') with semi-circular cross-section extending medially to laterally, and the two are aligned with one another, and
- a tibial part (10) which has two sliding tracks (11, 12) on which the runners (3, 4) of the femoral part (2) may execute a sliding movement, a web (13) extending in the femoral direction between the two sliding tracks (11, 12) and which engages through the slot (7) of the femoral part (2) and is guided closely adjacent the inner surfaces thereof, as well as a cylindrical guide bolt (14) extending medially to laterally in the upper region of the web (13) and which lies in the recesses (9 or 9') of the webs (8) in the femoral part (2) and is secured in this position by parts (17) of the stem part (1) surrounding it.

2. Knee joint endoprosthesis according to claim 1, in which the femoral part (2) consists of metal and the parts contacting it: sliding tracks (11, 12), web (13) and guide bolt (14) of tibial part (10) have surfaces of low-wear plastic.

3. Knee joint endoprosthesis according to claim 1, in which web (13) is reinforced by means of a metal insert (16).

4. Knee joint endoprosthesis according to claim 2 or 3, in which guide bolt (14) is reinforced by means of an inner metal core (15).

5. Knee joint endoprosthesis according to one of claims 2 to 4, in which the sliding tracks (11, 12) and the surface of web (13) are shaped to be integral in a single attachment piece, which is connected to the otherwise metallic tibial part (10).

6. Knee joint endoprosthesis according to claim 3, in which metal insert (16) of the web (13) is designed to be integral with the metallic parts of the tibial part (10).

7. Knee endoprosthesis according to one of claims 1 to 6, in which the recesses (9, 9') have a depth (T) which corresponds essentially to half the diameter (D) of guide bolt (14).

## Revendications

1. Endoprothèse de l'articulation du genou constituée par
- une partie de fémur (2) pouvant être raccordée à une partie de tige (1) à ancrer dans l'os du fémur, partie de tige qui est munie de deux patins de coulissement (3, 4), qui sont reliés entre eux ventralement par un pontet (6) portant un élément de liaison 5 pour la partie de tige (1) et délimitent entre eux une fente (7) s'étendant de la partie ventrale vers la partie dorsale, fente qui de chaque côté en direction du fémur est chaque fois bordée par une aile (8 ou 8'), dans laquelle il est prévu chaque fois une gorge s'étendant de la partie médiane vers la partie latérale (9 ou 9') avec une section transversale de cercle primitif, qui sont toutes deux en alignement l'une sur l'autre, et
- une partie de tibia (10), qui s'engage sur deux glissières (11, 12) sur lesquelles les patins de coulissement (3, 4) de la partie de fémur (2) peuvent effectuer un mouvement coulissant, une aile (13) dirigée vers le fémur entre les deux glissières (11, 12), qui s'engage par la fente (7) de la partie de fémur (2) et est amenée étroitement en appui sur ses surfaces internes, ainsi qu'un axe de guidage (14) cylindrique s'étendant de la partie médiane vers la partie latérale dans la zone supérieure de l'aile (13), qui repose dans les gorges (9 ou 9') des ailes (8) dans la partie de fémur (2) et dans cette position est assujetti en place par des éléments (17) de la partie de tige (1) qui l'enserrent.

2. Endoprothèse pour l'articulation du genou selon la revendication 1, dans laquelle la partie de fémur (2) est réalisée en métal et les parties qui viennent en contact avec celle-ci : glissières (11, 12), aile (13) et axe de guidage (14) de la partie de tibia (10) présentent des surfaces en une matière synthétique à faible coefficient de friction.

3. Endoprothèse pour l'articulation du genou selon la revendication 1, dans laquelle l'aile (13) est renforcée par un insert métallique (16).

4. Endoprothèse pour l'articulation du genou selon la revendication 2 ou 3, dans laquelle l'axe de guidage (14) est renforcé par un noyau métallique interne (15).

5. Endoprothèse pour l'articulation du genou selon l'une des revendications 2 à 4, dans laquelle les glissières (11, 12) et la surface de l'aile (13) sont réalisées de façon monobloc en un seul élément qui est raccordé à la partie de tibia (10) pour le reste métallique.

6. Endoprothèse pour l'articulation du genou selon la revendication 3, dans laquelle l'insert métallique (16) de l'aile (13) est réalisé d'une seule pièce avec les éléments métalliques de la partie de tibia (10).

7. Endoprothèse pour l'articulation du genou selon l'une des revendications 1 à 6, dans laquelle les gorges (9, 9') présentent une profondeur (T) qui correspond essentiellement au rayon (D) de l'axe de guidage (14).
